# EUROPEAN PATENT APPLICATION

(11) **EP 4 169 864 A1**
(43) Date of publication of application: **26.04.2023**
(21) Application number: 21208245.7
(22) Date of filing: 15.11.2021
(51) Int. Cl.: B66B 31/02

(54) **HANDRAIL BELT DISINFECTION MACHINE FOR ESCALATORS**

(30) Priority: 20.10.2021 CN 202111221066
(71) Applicant: Wang, Yi, Shanghai (CN)
(72) Inventor: Wang, Yi, Shanghai (CN)
(74) Representative: Zaboliene, Reda

(57) **Abstract**

A handrail belt disinfection machine for escalators includes a base and a rolling device having a first support arm and a second support arm on both sides of the base respectively, and both sides of the rolling device are connected to the first support arm and the second support arm respectively, and the rolling device has a disinfection device and drives the operation of the disinfection device. During the use of this machine with novel structure and clever design, the rolling device is contacted with a handrail belt of an escalator, and the movement of the handrail belt drives the rolling device to rotate and further drive the disinfection device to disinfect the handrail belt. When the escalator is not in use, the disinfection device will stop working, so as to achieve energy saving and environmental protection effects.

## Description

### FIELD OF THE INVENTION

The present invention relates to the field of cleaning and disinfecting equipment, and more particularly to a handrail belt disinfection machine for escalators.

### BACKGROUND OF THE INVENTION

Escalator is a loop moving staircase used as a fixed electric driving equipment for carrying people slantingly up and down between floors, which is often used in public places such as subway, shopping mall, etc. At present, the escalator (especially the handrail belt of the escalator) is cleaned and disinfected manually, and there are not too many disinfection devices for the handrail belt of the escalator, and most of these disinfection devices are fixedly connected to a part of the escalator, which involves the issue of relevant safety certification of the escalator. In addition, the installation, removal and maintenance of these disinfection devices are generally done by professional technicians, and are difficult to ordinary janitors, and these problems hinder the popularity of large-scale applications of the automatic disinfection devices of the handrail belt.

### SUMMARY OF THE INVENTION

In view of the aforementioned drawbacks of the prior art, the present invention provides a handrail belt disinfection machine for escalators with the features of energy saving, simple operation, and convenient installation.

To overcome the drawbacks of the prior art, the present invention discloses a handrail belt disinfection machine for escalators including a base and a rolling device, and a first support arm and a second support arm installed to both ends of the base respectively, and both ends of the rolling device being coupled to the first support arm and the second support arm respectively, and the rolling device having a disinfection device installed thereon, for driving the operation of the disinfection device.

Wherein, the rolling device is a roller, and both ends of the roller have a first connecting block and a second connecting block respectively, and the first support arm has a transmission device disposed on an outer side thereof and transmissively coupled to the first connecting block, and the second connecting block is movably coupled to the second support arm, and the transmission device is provided for driving the operation of the disinfection device.

Wherein, the base has a liquid storage tank disposed therein, and the liquid storage tank have a tank cover installed thereon, and the liquid storage tank has a liquid supply device installed at the outer periphery thereof, and the disinfection device comprises a water absorbent layer and a liquid outlet pipe, and the liquid storage tank and the liquid outlet pipe are communicated with the liquid supply device, and the roller has a through hole formed at a middle part thereof, and the liquid outlet pipe is extended into the through slot, and the roller has a plurality of hollow slots formed on an outer wall thereof and communicated with the through slot, and the water absorbent layer is sheathed on the outer periphery of the roller.

Wherein, the first support arm has a through hole formed thereon, and the through hole has a bearing installed therein, and the transmission device comprises a first synchronous wheel, a second synchronous wheel, a synchronous belt and a connector, and the connector is passed and installed to the bearing, and the connector has a third connecting block disposed at an end thereof and coupled to the first connecting block, and the other end of the connector is coupled to the first synchronous wheel, and the first synchronous wheel and the second synchronous wheel are transmissively coupled to each other through the synchronous belt, and provided for driving the operation of the disinfection device.

Wherein, the transmission device has an output shaft installed at an output end thereof, and the liquid supply device comprises a first peristaltic pump and a second peristaltic pump coupled to the output shaft, and the first peristaltic pump has a first connecting end and a second connecting end, and the second peristaltic pump has a third connecting end and a fourth connecting end, and the first connecting end and the fourth connecting end are communicated with the liquid storage tank, and the liquid outlet pipe comes with a quantity of two, and the two liquid outlet pipes are communicated with the second connecting end and the third connecting end respectively, and the first peristaltic pump and the second peristaltic pump are provided for sending a disinfectant to the roller through the liquid outlet pipe and sending air into the liquid storage tank through the liquid outlet pipe.

Wherein, the first peristaltic pump and the second peristaltic pump are bi-directional peristaltic pumps, and the roller is installed at a middle part of the base, and both front end rear ends of the base have a guiding arc-surface.

Wherein, a gearbox is installed between the output shaft and the first peristaltic pump and second peristaltic pump, and the first peristaltic pump and the second peristaltic pump are coupled to an output end of the gearbox.

Wherein, the first synchronous wheel has a size larger than the size of the second synchronous wheel.

Wherein, the first support arm has a protective cover installed to an outer periphery thereof, and the second support arm is detachably mounted onto the base.

Wherein, the base has a plurality of counterweights installed therein, a handle installed to the front end of the base, a moving inclined plane formed at the rear end of the base, and two or more universal wheels mounted onto the moving inclined plane.

The present invention has the following advantages and effects: The machine of this invention comes with a novel structure and a clever design, the rolling device is contacted with a handrail belt of an escalator, and the movement of the handrail belt drives the rolling device to rotate and further drive the disinfection device to disinfect the handrail belt. When the escalator is not in use, the disinfection device will stop working, so as to achieve energy saving and environmental protection effects. The device only performs the disinfection during the use of the escalator to effectively avoid unnecessary waste, and the operators just need to contact the rolling device with the handrail belt of the escalator to start the cleaning and disinfection, and thus the device is simple-to-operate and convenient-to-install.

### BRIEF DESCRIPTION OF THE DRAWINGS

FIG. 1 is a schematic view of the structure of the present invention;
FIG. 2 is a first exploded view of the structure of the present invention;
FIG. 3 is a second exploded view of the structure of the present invention;
FIG. 4 is a third exploded view of the structure of the present invention;
FIG. 5 is a schematic view of a liquid storage tank and a liquid supply device of the present invention;
FIG. 6 is a schematic view of a liquid supply device of the present invention; and
FIG. 7 is a schematic view showing the installation position of the present invention with an escalator.

### DESCRIPTION OF THE PREFERRED EMBODIMENTS

The technical contents of the present invention will become apparent with the detailed description of preferred embodiments accompanied with the illustration of related drawings as follows. It is intended that the embodiments and figures disclosed herein are to be considered illustrative rather than restrictive.

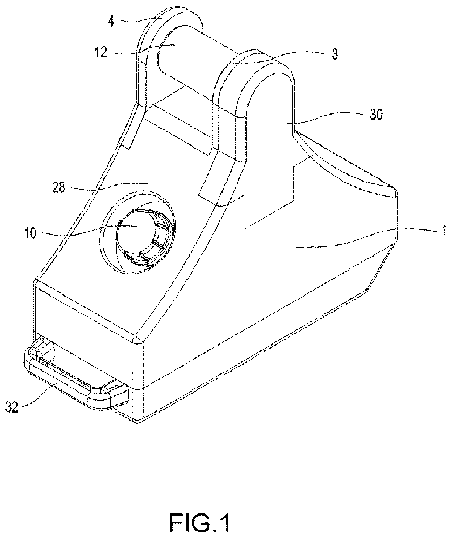

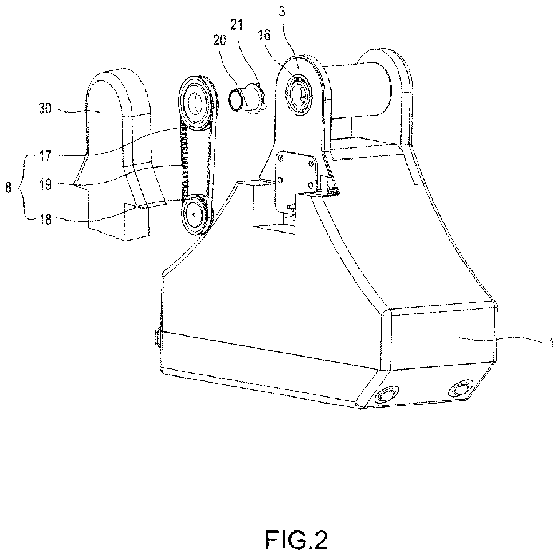

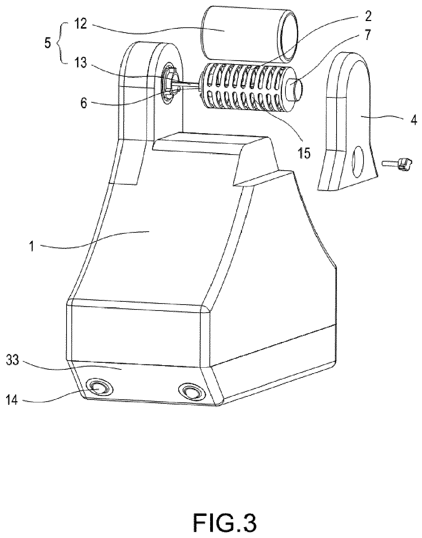

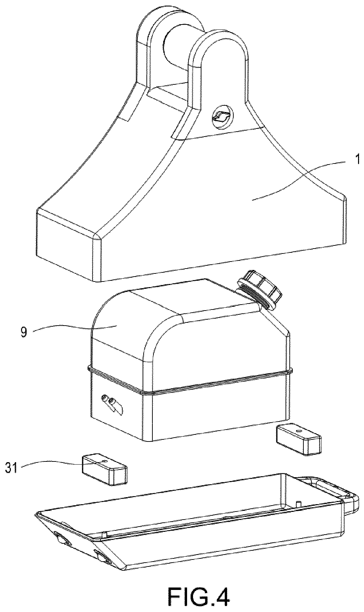

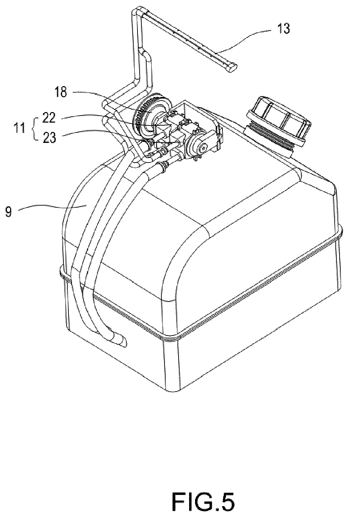

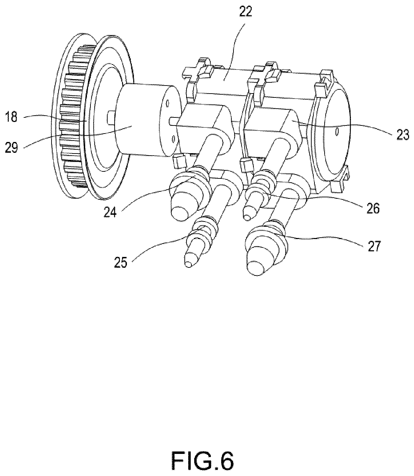

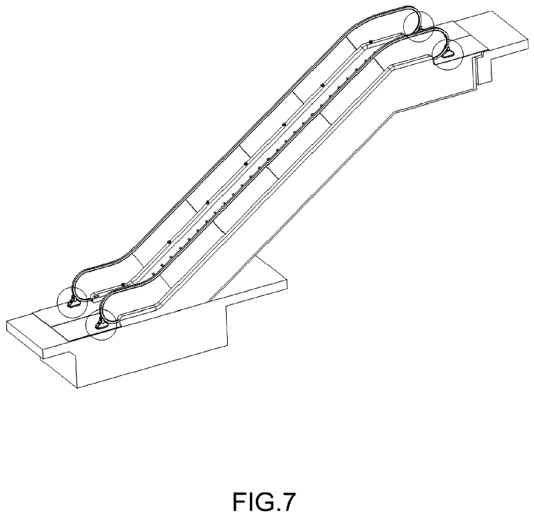

With reference to FIGS. 1 to 7 for a handrail belt disinfection machine for escalators, the machine includes a base 1 and a rolling device 2, and both ends of the base 1 have a first support arm 3 and a second support arm 4 respectively, and both ends of the rolling device 2 are connected to the first support arm 3 and the second support arm 4 respectively, and the rolling device 2 includes a disinfection device 5 and is provided for driving the operation of the disinfection device 5. Specifically, the present invention comes with a novel structure and a clever design. When use, the rolling device 2 is contacted with a handrail belt of an escalator, and the movement of the handrail belt drives the rolling device 2 to operate and further drive the disinfection device 5 to disinfect the handrail belt. When the escalator is not in use, the disinfection device 5 will also stop working, so that the machine can achieve the energy saving and environment protection effects. The disinfection only takes place when the escalator is in use, so as to effectively avoid unnecessary waste. In addition, the escalator still keeps operating during the disinfection, and the operation staff just needs to contact the rolling device 2 with the handrail belt of the escalator to start cleaning and disinfection, and thus the machine of this invention is simple-to-operate and convenient-to-install.

In an embodiment of the handrail belt disinfection machine for escalators, the rolling device 2 is a roller, and both ends of the roller have a first connecting block 6 and a second connecting block 7 respectively, and an outer side of the first support arm 3 has a transmission device 8, and the first connecting block 6 is transmissively connected to the transmission device 8, and the second connecting block 7 is movably connected to the second support arm 4 and the transmission device 8 is provided for driving the operation of the disinfection device 5. Specifically, the structural connection is stable.

In an embodiment of the handrail belt disinfection machine for escalators, the base 1 has a liquid storage tank 9, and a tank cover 10 is installed to the liquid storage tank 9, and a liquid supply device 11 is installed at the outer periphery of the liquid storage tank 9. The disinfection device 5 includes a water absorbent layer 12 and a liquid outlet pipe 13, and the liquid storage tank 9 and the liquid outlet pipe 13 are communicated with the liquid supply device 11, and a through slot is formed at the middle part of the roller, and the liquid outlet pipe 13 is extended into the through slot. An outer wall of the roller has a plurality of hollow slots 15 communicated with the through slot, and the water absorbent layer 12 is sheathed on the outer periphery of the roller. Specifically, the liquid supply device 11 sends a disinfectant stored in the liquid storage tank 9 to the liquid outlet pipe 13 and sprays the disinfectant of the liquid outlet pipe 13 onto the water absorbent layer 12, and the water absorbent layer 12 is in contact with the handrail belt of the escalator to achieve the cleaning and disinfection effects. Further, the liquid storage tank 9 adopts a fully sealed design, so that even if the handrail belt disinfection machine is rolled over, the disinfectant will not leak out, so as to prevent damages to the escalator by the disinfectant, and the water absorbent layer 12 is made of a soft absorbent material such as sponge.

In an embodiment of the handrail belt disinfection machine for escalators, the first support arm 3 has a through hole, and a bearing 16 installed in the through hole. The transmission device 8 includes a first synchronous wheel 17, a second synchronous wheel 18, a synchronous belt 19 and a connector 20, and the connector 20 is passed and installed to the bearing 16, and an end of the connector 20 has a third connecting block 21 connected to the first connecting block 6, and the other end of the connector 20 is connected to the first synchronous wheel 17, and the first synchronous wheel 17 is transmissively connected to the second synchronous wheel 18 through the synchronous belt 19, and the second synchronous wheel 18 is provided for driving the operation of the disinfection device 5. Specifically, the installed structure is stable and the transmission effect is good. Further, the first connecting block 6 includes a plurality of snap slots formed thereon, and the third connecting block 21 has a plurality of snap blocks protruded therefrom, and the snap slots and the snap blocks arc configured to be corresponsive to one another. Further, both of the connector 20 and the first synchronous wheel 17 have a round hole, and the liquid outlet pipe 13 is passed through the round hole and extended into the through slot.

In an embodiment the handrail belt disinfection machine for escalators, an output end of the transmission device 8 has an output shaft, and the liquid supply device 11 includes a first peristaltic pump 22 and a second peristaltic pump 23, and both of the first peristaltic pump 22 and the second peristaltic pump 23 are connected to the output shaft. The first peristaltic pump 22 has a first connecting end 24 and a second connecting end 25, and the second peristaltic pump 23 has a third connecting end 26 and a fourth connecting end 27, and both of the first connecting end 24 and the fourth connecting end 27 are communicated with the liquid storage tank 9, wherein there are two liquid outlet pipes 13 communicated with the second connecting end 25 and the third connecting end 26 respectively, and the first peristaltic pump 22 and the second peristaltic pump 23 are provided for sending a disinfectant into the roller through the liquid outlet pipe 13 and sending air into the liquid storage tank 9 through the liquid outlet pipe 13. Specifically, the liquid storage tank 9 adopts a fully sealed design, so that when the disinfectant is drawn out, there will be a change of pressure in the liquid storage tank, which will affect the normal operation, so that the output end and input end of the first peristaltic pump 22 and the second peristaltic pump 23 are configured to be opposite in direction with each other, so that when the first peristaltic pump 22 or the second peristaltic pump 23 sends the disinfectant to the roller through the liquid outlet pipe 13, the other peristaltic pump will send air into the liquid storage tank 9 through the liquid outlet pipe 13. As a result, the present invention can be operated normally without being affected.

In an embodiment of the handrail belt disinfection machine for escalators, both of the first peristaltic pump 22 and the second peristaltic pump 23 are bi-directional peristaltic pumps, and the roller is installed at the middle part of the base 1, and both front and rear ends of the base 1 have a guiding arc-surface 28. Specifically, the handrail belt disinfection machine can be extended into the escalator from both ends, and both of the first peristaltic pump 22 and the second peristaltic pump 23 are bi-directional peristaltic pumps, so that the disinfection device 5 will not be affected by the forward or reverse rotation of the roller. In a clockwise rotation, the first peristaltic pump 22 sends the disinfectant from the liquid storage tank 9 into the roller and the second peristaltic pump 23 sends air from the roller into the liquid storage tank 9. In a counterclockwise rotation, the second peristaltic pump 23 sends the disinfectant from the liquid storage tank 9 into the roller and the first peristaltic pump 22 sends air from the roller into the liquid storage tank 9.

In an embodiment of the handrail belt disinfection machine for escalators, a gearbox 29 is installed between the output shaft and the first peristaltic pump 22 and the second peristaltic pump 23, and both of the first peristaltic pump 22 and the second peristaltic pump 23 are connected to the output end of the gearbox 29. Specifically, the gearbox 29 can be accelerated by the shifting of the internal gears.

In an embodiment of the handrail belt disinfection machine for escalators, the first synchronous wheel 17 has a size larger than the size of the second synchronous wheel 18. Specifically, this setup achieves the accelerated transmission effect.

In an embodiment of the handrail belt disinfection machine for escalators, a protective cover 30 is installed at the outer periphery of the first support arm 3, and the second support arm 4 is detachably installed on the base 1. Specifically, this setup is convenient to install, maintain and change components, wherein the components can be replaced to extend the machine service life, reduce the cost of use, and achieve the environmental protection effect.

In an embodiment of the handrail belt disinfection machine for escalators, the base 1 includes a plurality of counterweights 31 installed therein, and the front end of the base 1 has a handle 32, and the rear end of the base 1 has a moving inclined plane 33, and two or more universal wheels 14 are mounted onto the moving inclined plane 33. Specifically, this setup allows users to conveniently lift the machine and drag the machine after the universal wheels 14 have landed the floor. This machine can be transferred to another escalator when the cleaning and disinfection of one escalator has been done for a period of time, and thus this machine can be used for cleaning and disinfection a multiple of escalators. In the meantime, the counterweight 31 improves the safety and effectively reduce the random sliding of the base 1. Further, this escalator disinfection machine only touches the escalator through the roller, but will not touch other parts of the escalator, so that the machine will remain unmoved by its own weight during operation. If a pedestrian touches the machine, the roller of the machine will be separated from the escalator handrail belt automatically. Therefore, the machine of this invention is safe and capable of preventing pinches, scratches, or injuries.

While the invention has been described by way of example and in terms of a preferred embodiment, it is to be understood that the invention is not limited thereto. To the contrary, it is intended to cover various modifications and similar arrangements and procedures, and the scope of the appended claims therefore should be accorded the broadest interpretation so as to encompass all such modifications and similar arrangements and procedures.

## Claims

1. A handrail belt disinfection machine for escalators, comprising: a base and a rolling device, and a first support arm and a second support arm installed to both ends of the base respectively, and both ends of the rolling device being coupled to the first support arm and the second support arm respectively, and the rolling device having a disinfection device installed thereon, for driving the operation of the disinfection device.

2. The handrail belt disinfection machine for escalators according to claim 1, wherein the rolling device is a roller, and both ends of the roller have a first connecting block and a second connecting block respectively, and the first support arm has a transmission device disposed on an outer side thereof and transmissively coupled to the first connecting block, and the second connecting block is movably coupled to the second support arm, and the transmission device is provided for driving the operation of the disinfection device.

3. The handrail belt disinfection machine for escalators according to claim 2, wherein the base has a liquid storage tank disposed therein, and the liquid storage tank have a tank cover installed thereon, and the liquid storage tank has a liquid supply device installed at the outer periphery thereof, and the disinfection device comprises a water absorbent layer and a liquid outlet pipe, and the liquid storage tank and the liquid outlet pipe are communicated with the liquid supply device, and the roller has a through hole formed at a middle part thereof, and the liquid outlet pipe is extended into the through slot, and the roller has a plurality of hollow slots formed on an outer wall thereof and communicated with the through slot, and the water absorbent layer is sheathed on the outer periphery of the roller.

4. The handrail belt disinfection machine for escalators according to claim 3, wherein the first support arm has a through hole formed thereon, and the through hole has a bearing installed therein, and the transmission device comprises a first synchronous wheel, a second synchronous wheel, a synchronous belt and a connector, and the connector is passed and installed to the bearing, and the connector has a third connecting block disposed at an end thereof and coupled to the first connecting block, and the other end of the connector is coupled to the first synchronous wheel, and the first synchronous wheel and the second synchronous wheel are transmissively coupled to each other through the synchronous belt, and provided for driving the operation of the disinfection device.

5. The handrail belt disinfection machine for escalators according to claim 3, wherein the transmission device has an output shaft installed at an output end thereof, and the liquid supply device comprises a first peristaltic pump and a second peristaltic pump coupled to the output shaft, and the first peristaltic pump has a first connecting end and a second connecting end, and the second peristaltic pump has a third connecting end and a fourth connecting end, and the first connecting end and the fourth connecting end are communicated with the liquid storage tank, and the liquid outlet pipe comes with a quantity of two, and the two liquid outlet pipes are communicated with the second connecting end and the third connecting end respectively, and the first peristaltic pump and the second peristaltic pump are provided for sending a disinfectant to the roller through the liquid outlet pipe and sending air into the liquid storage tank through the liquid outlet pipe.

6. The handrail belt disinfection machine for escalators according to claim 5, wherein the first peristaltic pump and the second peristaltic pump are bi-directional peristaltic pumps, and the roller is installed at a middle part of the base, and both front end rear ends of the base have a guiding arc-surface.

7. The handrail belt disinfection machine for escalators according to claim 5, further comprising a gearbox installed between the output shaft and the first peristaltic pump and second peristaltic pump, and the first peristaltic pump and the second peristaltic pump are coupled to an output end of the gearbox.

8. The handrail belt disinfection machine for escalators according to claim 3, wherein the first synchronous wheel has a size larger than the size of the second synchronous wheel.

9. The handrail belt disinfection machine for escalators according to claim 1, wherein the first support arm has a protective cover installed to an outer periphery thereof, and the second support arm is detachably mounted onto the base.

10. The handrail belt disinfection machine for escalators according to claim 1, wherein the base has a plurality of counterweights installed therein, a handle installed to the front end of the base, a moving inclined plane formed at the rear end of the base, and two or more universal wheels mounted onto the moving inclined plane.
